**Europäisches Patentamt**

**European Patent Office**

**Office européen des brevets**

(19)

(11) Numéro de publication : **0 185 559 B2**

(12)

# NOUVEAU FASCICULE DE BREVET EUROPEEN

(45) Date de publication du nouveau fascicule du brevet : **25.03.92 Bulletin 92/13**

(51) Int. Cl.$^5$ : **C07C 17/08, C07C 17/04**

(21) Numéro de dépôt : **85401820.7**

(22) Date de dépôt : **19.09.85**

(54) **Procédé de préparation d'halogénures allyliques primaires et/ou tertiaires.**

Le dossier contient des informations techniques présentées postérieurement au dépôt de la demande et ne figurant pas dans le présent fascicule.

(30) Priorité : **20.09.84 FR 8414425**

(43) Date de publication de la demande : **25.06.86 Bulletin 86/26**

(45) Mention de la délivrance du brevet : **20.04.88 Bulletin 88/16**

(45) Mention de la décision concernant l'opposition : **25.03.92 Bulletin 92/13**

(84) Etats contractants désignés : **AT BE CH DE FR GB IT LI LU NL SE**

(56) Documents cités :
**EP-A- 0 132 544**
**DE-A-17 685 44**
**US-A- 3 055 954**
**US-A- 3 812 212**
**US-A- 4 168 271**

(73) Titulaire : **RHONE-POULENC SANTE**
**20, avenue Raymond Aron**
**F-92160 Antony (FR)**

(72) Inventeur : **Mulhauser, Michel**
**Immeuble "Frênes 4" Résidence Charrière Blanche**
**F-69130 Ecully (FR)**

(74) Mandataire : **Le Pennec, Magali et al**
**RHONE-POULENC SANTE, Service Brevets,**
**20 Avenue Raymond Aron**
**F-92165 Antony Cédex (FR)**

EP 0 185 559 B2

Jouve, 18, rue Saint-Denis, 75001 PARIS

## Description

La présente invention concerne un nouveau procédé de préparation d'halogénures allyliques primaires et/ou tertiaires par action d'un hydracide halogéné sur un diène conjugué terminal en présence d'un catalyseur constitué d'un sel cuivreux associé à un sel d'ammonium quaternaire ou à un sel de phosphonium.

L'art antérieur enseigne de nombreux procédés d'hydrohalogénation de diènes conjugués qui présentent l'inconvénient majeur d'être peu sélectifs.

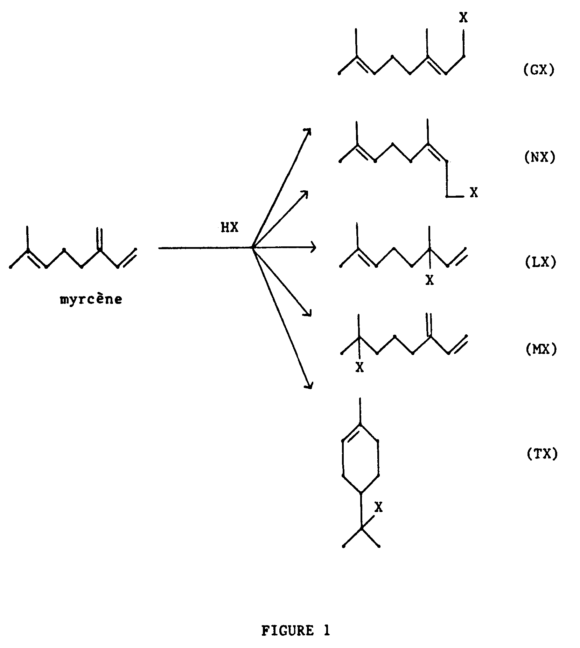

Plus particulièrement, l'hydrohalogénation du myrcène peut conduire à un mélange d'halogénures selon le schéma représenté figure 1 dans lequel

X représente un atome de chlore ou de brome,
GX est l'halogénure de géranyle
NX est l'halogénure de néryle
LX est l'halogénure de linalyle
MX est l'halogénure de myrcényle, et
TX est l'halogénure de terpényle.

Le mélange de ces différents halogénures dans des proportions variables est appelé communément bromhydrate ou chlorhydrate de myrcène.

Selon le brevet américain US 3 031 442, l'hydrochloration du myrcène pur en présence de chlorure cuivreux conduit à un mélange des chlorures de géranyle et de néryle (75 à 80%), de linalyle (5 à 10%) et de terpényle (10 à 15%).

L'hydrochloration du myrcène à 75,8% de pureté en présence de chlorure mercurique conduit à un mélange des chlorures de géranyle et de néryle (2 %), de linalyle (5%), de myrcényle (77%) et de terpényle (16%) en opérant dans les conditions décrites dans le brevet américain US 3 413 364.

D'après le brevet américain US 3 016 408, l'hydrochloration ou l'hydrobromation du myrcène technique en présence d'acétate cuivrique conduit à un mélange d'halogénures de géranyle et de néryle (30 à 35%), de linalyle (40 à 49%), de myrcényle (0 à 10%) et de terpényle (15 à 18 %).

Dans la demande de brevet européen EP 132 544, publiée le 13 février 1985, est décrite l'hydrohalogénation d'un diène conjugué, en particulier du myrcène, en présence d'un catalyseur à base de cuivre et d'un sel d'ammonium quaternaire ou d'un sel de phosphonium contenant au moins 18 atomes de carbone. Le sel d'ammonium quaternaire ou le sel de phosphonium permet la solubilisation du catalyseur dans le myrcène. Cependant il est difficile de séparer le système catalytique des produits de la réaction du fait que les sels d'ammonium quaternaire et les sels de phosphonium tels qu'ils sont définis dans la demande de brevet européen EP 132 544 ne peuvent pas être facilement éliminés par exemple par lavage à l'eau du mélange réactionnel.

Compte tenu de l'intérêt du myrcène dans la synthèse des vitamines A et E et de produits terpéniques tels que le citral ou l'acétate de linalyle, il est particulièrement intéressant de disposer d'un procédé sélectif qui conduise essentiellement aux halogénures de géranyle, de néryle et de linalyle qui, étant donné leur fragilité, peuvent être séparés facilement du mélange réactionnel.

Il a maintenant été trouvé, et c'est ce qui fait l'objet de l'invention un procédé de préparation d'halogénures allyliques primaires et/ou tertiaires par action d'un hydracide halogéné sur un diène conjugué terminal comportant des enchaînements terpéniques en présence d'un catalyseur constitué d'halogénure cuivreux tel que le chlorure ou l'iodure cuivreux associé à un sel d'ammonium quaternaire ou à un sel de phosphonium contenant au maximum 16 atomes de carbone ou à un sel d'amine tertiaire contenant au maximum 10 atomes de carbone caractérisé en ce que l'on effectue la réaction à une température inférieure à 20°C dans un solvant organique capable de solubiliser le système catalytique, puis traite le mélange réactionnel par l'eau et un solvant organique, afin d'éliminer le système catalytique en solution aqueuse, puis sépare les halogénures allyliques primaires et/ou tertiaires après élimination du solvant de la phase organique, et les purifie éventuellement par distillation.

Généralement le procédé est mis en oeuvre à une température inférieure à 0°C.

Par ailleurs, pour éviter la formation de produits polyhalogénés, il est particulièrement avantageux d'utiliser l'hydracide halogéné en quantité stoechiométrique par rapport au diène conjugué mis en oeuvre.

Généralement, pour la mise en oeuvre du procédé selon l'invention, on utilise un halogénure cuivreux dans un rapport molaire compris entre 0,05 et 10%, de préférence entre 0,1 et 3%, par rapport au diène conjugué mis en oeuvre associé à un sel d'ammonium quaternaire ou un sel de phosphonium dans un rapport molaire compris entre 0,05 et 10 %, de préférence entre 0,1 et 3% par rapport au diène conjugué mis en oeuvre.

Les sels d'ammonium quaternaire et les sels de phosphonium sont choisis parmi les halogénures de tétraalcoylammonium ou de tétraalcoylphosphonium contenant au maximum 16 atomes de carbone tels que les chlorures ou bromures de tétra-n.butylammonium ou tétra-n. butylphosphonium. Les sels d'amines tertiaires sont choisis parmi les halohydrates de trialcoylamines contenant au maximum 10 atomes de carbone tels que le chlorhydrate de triéthylamine.

Le mélange des halogénures allyliques primaires et/ou tertiaires obtenus selon le procédé de la présente invention peut être séparé du milieu réactionnel après lavage à l'eau, pour éliminer le système catalytique en solution en phase aqueuse, et par un solvant organique choisi parmi les hydrocarbures aliphatiques (pentane, hexane) ou aromatiques (benzène). Les produits d'hydrohalogénation sont obtenus après évaporation de la phase organique et ils peuvent être purifiés par distillation.

En particulier, dans le cas du myrcène, le procédé selon l'invention permet d'obtenir les chlorures de géranyle et de néryle avec des rendements généralement supérieurs à ceux obtenus selon les procédés antérieurement connus.

Le procédé selon la présente invention convient particulièrement bien pour réaliser l'hydrochloration des diènes conjugués terminaux comportant des enchaînements terpéniques tels que le myrcène, le ß-farnésène, le ß-springène ou le triméthyl-7,11,15 méthylène-3 chloro- 15 hexadécatriène- 1, 6, 10 (ou chloro- 15 ß-springène) dont les halogénures allyli ques peuvent être transformés, par exemple en vitamine E, selon les méthodes connues décrites dans la littérature.

Les exemples suivants, donnés à titre non limitatif, montrent comment l'invention peut être mise en pratique.

*Exemple 1*

Dans un ballon tricol de 250 cm³ muni d'un agitateur magnétique, d'un thermomètre, d'un tube plongeant au niveau de l'agitateur et d'une tête à hydrogéner, on introduit, sous atmosphère d'argon, 1,9 g de chlorure de tétra-n.butylammonium (0,00685 mole) qui est séché par chauffage à une température comprise entre 90 et 100°C pendant 2 heures sous pression réduite. Après refroidissement à 40°C, on introduit rapidement 75 cm³ de chlorure de méthylène anhydre et 0,675 g de chlorure cuivreux (0,00682 mole). On agite, sous atmosphère d'argon, jusqu'à dissolution du chlorure cuivreux. On obtient ainsi une solution homogène jaune qui est refroidie à une température comprise entre -3 et -5°C. On ajoute alors 37,2 g de myrcène dont la pureté est supérieure à 95% (0,274 mole) puis on ajoute 10 g d'acide chlorhydrique sec (0,274 mole) en 3 heures 15 minutes. La solution homogène devient brune. Le mélange réactionnel est versé dans 200 cm³ d'une solution aqueuse de chlorure d'ammonium à 100 g/litre. La phase organique est séparée puis on extrait la phase aqueuse par 100 cm³ de chlorure de méthylène. Les phases organiques réunies sont lavées par 3 fois 50 cm³ d'eau puis séchées sur carbonate de potassium. Après filtration et évaporation du solvant, le résidu obtenu est repris par 100 cm³ de pentane: le chlorure de tétra-n-butylammonium précipite. Après filtration, le solvant est évaporé sous pression réduite. On obtient, avec un rendement de 90,4%, 42,7 g d'une huile légèrement jaune dont l'analyse par chromatographie en phase vapeur (CPV) et par résonance magnétique nucléaire (RMN) montre qu'elle est constituée de:

|  | CPV | RMN |
|---|---|---|
| – chlorures de géranyle et de néryle | 85,4% | 87% |
| – chlorure de linalyle | 5,4% | 6% |
| – chlorure de myrcényle | 0,2% |  |
| – chlorure de terpényle | 2 % | 3% |
| – myrcène | 3,3% | 4% |
| – hydrocarbures en $C_{10}$ dihydrochlorés | 2,1% |  |
| – hydrocarbures en $C_{10}$ (en dehors du myrcène) | 1 % |  |

Le taux de transformation du myrcène est voisin de 97% et le rendement en chlorures de géranyle, de néryle et de linalyle est voisin de 77,5%.

*Exemple 2*

Dans un ballon tricol de 500 cm³ muni d'une agitation magnétique, d'un thermomètre et d'un tube plongeant au niveau de l'agitateur et d'une tête à hydrogéner, on introduit, sous atmosphère d'argon, 2,01 g de chlorhydrate de triéthylamine, 100 cm³ de chlorure de méthylène et 1,45 g do chlorure cuivreux. On agite à une température de 20°C jusqu'à dissolution du chlorure cuivreux puis on refroidit à -10°C. On ajoute alors 100 g de myrcène dont la pureté est supérieure à 95% puis, en 2 heures, 26,8 g d'acide chlorhydrique anhydre en maintenant la température entre -9 et -11°C.

La solution obtenue est versée dans 200 cm$^3$ d'une solution aqueuse de chlorure d'ammonium à 10%. Les produits organiques sont extraits par 200 cm$^3$ de pentane. La phase organique est lavée à l'eau puis séchée sur carbonate de potassium. Après évaporation des solvants sous pression réduite, on obtient 128,1 g d'une huile dont l'analyse par chromatographie en phase vapeur montre qu'elle est constituée de:

| | |
|---|---|
| – chlorure de géranyle et de néryle | 81,5% |
| – chlorure de linalyle | 6,15% |
| – chlorure de terpényle | 1,2 % |
| – myrcène | 0,95% |
| – hydrocarbures en $C_{10}$ dihydrochlorés | 1,7 % |

Le taux de transformation du myrcène est voisin de 99%.

*Exemple 3*

En opérant comme dans l'exemple 2 mais en utilisant 1 mole de myrcène et en ajoutant l'acide chlorhydrique à une température comprise entre - 14 et -16°C, on obtient 173,6 g d'une huile dont la composition est la suivante:

| | |
|---|---|
| – chlorures de géranyle et de néryle | 86,8% |
| – chlorure de linalyle | 5,9% |
| – chlorure de myrcényle | 0,2% |
| – chlorure de terpényle | 1,6% |
| – myrcène | 1,6% |
| – hydrocarbures en $C_{10}$ dihydrochlorés | 1,1% |
| – hydrocarbures en $C_{10}$ (en dehors du myrcène | 2,3% |

Le taux de transformation du myrcène est voisin de 98,4% et le rendement en chlorures de géranyle, de néryle et de linalyle est de 85,2%.

*Exemple 4*

On opère comme dans l'exemple 2 mais en utilisant un rapport molaire de chlorure cuivreux et de chlorure de tétra-n.-butylammonium de 0, 5% par rapport au myrcène mis en oeuvre (au lieu de 2,5%) et en ajoutant l'acide chlorhydrique sec à une température comprise entre -14 et - 16°C. On obtient une huile dont la composition est la suivante:

| | |
|---|---|
| – chlorures de géranyle et de néryle | 71 % |
| – chlorure de linalyle | 11,8% |
| – chlorure de myrcényle | 0,4% |
| – chlorure de terpényle | 4,1% |
| – myrcène | 7 % |
| – hydrocarbures en $C_{10}$ dihydrochlorés | 1,3% |
| – hydrocarbures en $C_{10}$ (en dehors du myrcène) | 3,5% |

*Exemple 5* On opère comme dans l'exemple 2 en utilisant:

| | | |
|---|---|---|
| – myrcène | 37,24 g | (0,274 mole) |
| – acide chlorhydrique sec | 10 g | (0,274 mole) |
| – chlorure cuivreux | 2,7 g | (0,0274 mole) |
| – chlorure de tétra-n. butylammonium | 7,6 g | (0,0274 mole) |
| – chlorure de méthylène | 75 cm$^3$ | |

L'acide chlorhydrique sec et ajouté en 30 minutes à une température comprise entre -3 et -5°C.

Après traitement du mélange réactionnel, on obtient 47,42 g d'une huile dont la composition est la suivante:

| | CPV | RMN |
|---|---|---|
| – chlorures de géranyle et de néryle | 82,3% | 84% |
| – chlorure de linalyle | 4,7% | 6% |
| – chlorure de terpényle | 3,4% | 6% |
| – myrcène | 2,3% | 4% |
| – hydrocarbures en C$_{10}$ hydro- chlorés | 0,5% | |
| – hydrocarbures en C$_{10}$ dihydrochlorés | 2,8% | |
| – hydrocarbure en C$_{10}$ (en dehors du myrcène) | 3,6% | |

## Exemple 6

Dans un appareil identique à celui décrit dans l'exemple 2, on introduit, sous atmosphère d'argon, 1,4 g de chlorhydrate de triéthylamine (0,01 mole; 2,5% molaire par rapport au myrcène mis en oeuvre) et 120 cm$^3$ de chlorure de méthylène. On ajoute ensuite 1 g de chlorure cuivreux (0,01 mole; 2,5% molaire par rapport au myrcène mis en oeuvre). On agite jusqu'à l'obtention d'une solution jaune homogène qui est refroide à -5°C. On ajoute 56 g de myrcène dont la pureté est supérieure à 95% (0,41 mole) puis 15 g d'acide chlorhydrique sec (0,41 mole) en 5 heures. Après traitement du mélange réactionnel dans les conditions de l'exemple 2, on obtient 68,8 g d'une huile dont la composition est la suivante:

| | |
|---|---|
| – chlorures de géranyle et de néryle | 88,1% |
| – chlorure de linalyle | 5,1% |
| – chlorure de terpényle | 1,9% |
| – myrcène | 3,7% |
| – hydrocarbures en C$_{10}$ hydrochlorés | 0,2% |
| – hydrocarbures en C$_{10}$ dihydrochlorés | 0,4% |
| – hydrocarbures en C$_{10}$ (en dehors du myrcène) | 0,6% |

Le taux de transformation du myrcène est de 97% et le rendement en chlorures de géranyle, néryle et linalyle isolés est de 88%.

## Exemple 7

On opère comme dans l'exemple 6 mais en utilisant du myrcène technique dont la composition est la suivante:

| | |
|---|---|
| – myrcène | 68,3% |
| – β-pinène | 6,8% |
| – limonène | 9,3% |
| – autres hydrocarbures en $C_{10}$ | 7,9% |

Dans une solution de 103,8 g de myrcène technique dans 160 cm³ de chlorure de méthylène contenant un catalyseur constitué d'un mélange équimoléculaire de chlorure cuivreux et de chlorhydrate de triéthylamine (2,5% molaire par rapport au myrcène), on introduit, en 4 heures 30 minutes, à -5°C, 20 g d'acide chlorhydrique anhydre. Après traitement du mélange réactionnel, on obtient une huile dont l'analyse montre que:
– le taux de transformation du myrcène est de 92%
– le rendement en chlorures de géranyle, de néryle et de linalyle est de 91 % par rapport au myrcène transformé.

*Exemple 8*

Dans un appareil identique à celui décrit dans l'exemple 2, on introduit, sous atmosphère d'argon, 0,48 g de chlorhydrate de triéthylamine (10% molaire par rapport au β-springène), 15 cm³ de chlorure de méthylène, 10 cm³ d'acide acétique et 90 mg de chlorure cuivreux (2,5% molaire par rapport au β-springène). On agite le mélange réactionnel jusqu'à l'obtention d'une solution homogène. On refroidit à -10°C puis on ajoute 10 g (0,0367 mole) de β-springène (obtenu par condensation du chlorure de géranylmagnésium sur le chloro-3 myrcène dans les conditions décrites dans le brevet américain US 4 292 459) et, en 1 heure, 1, 3 g d'acide chlorhydrique gazeux sec. Après traitement du mélange réactionnel dans les conditions décrites précédemment, on obtient 10,9 g d'une huile jaune pâle (96,5% de la théorie) dont les spectres de masse et de résonance magnétique nucléaire du proton sont en accord avec la structur du produit attendu.

On soumet 2 g de cette huile à une hydrogénation catalytique en présence de 200 mg de palladium sur noir à 10% de palladium en opérant dans 20 cm³ d'éthanol sous une pression de 50 bars pendant 4 heures.

Le chromatogramme, par chromatographie en phase gazeuse capillaire du produit obtenu, est identique à celui du produit d'hydrogénation du β-springène en phytane dans les mêmes conditions.

*Exemple 9*

On opère comme dans l'exemple 2 mais en utilisant:

| | |
|---|---|
| – myrcène (dont la pureté est supérieure à 95%) | 50 g (0,367 mole) |
| – chlorure cuivreux | 1,82 g (0,0184 mole) |
| – chlorhydrate de triéthylamine | 2,53 g (0,0250 mole) |
| – anhydride acétique | 66 cm³ |
| – acide acétique | 33 cm³ |

L'acide chlorhydrique (13,4 g; 0,367 mole) est ajouté en 3 heures à -10°C.

Après traitement du mélange réactionnel, on obtient une huile dont l'analyse par chromatographie capillaire en phase vapeur montre qu'elle est constituée de:

| | |
|---|---|
| – chlorures de géranyle et de néryle | 63,6% |
| – chlorure de linalyle | 5 % |
| – chlorure de myrcényle | 1 % |
| – chlorure de terpényle | 2,8% |
| – myrcène | 22,9% |
| – hydrocarbures en $C_{10}$ dihydrochlorés | 1,9% |
| – hydrocarbures en $C_{10}$ (en dehors du myrcène) | 2,6% |

6

Le taux de transformation du myrcène est de 81 % et le rendement en chlorures de géranyle, de néryle et le linalyle est de 89% par rapport au myrcène transformé.

*Exemple 10*

On opère comme dans l'exemple 8 mais en effectuant l'hydrochloration sur 6,3 g de triméthyl-7,11,15 méthylène-3 chloro-15 hexadécatriène-1,6,10 (ou chloro-15 β-springène) (0,02 mole). On obtient avec un rendement de 95%, le dichloro-1,15 tétraméthyl-3,7,11,15 hexadécatriène-2, 6, 10 sous forme du mélange des formes E et Z qui est hydrogéné en phytane avec une sélectivité de 98%.

Le triméthyl-7,11,15 méthylène-3 chloro-15 hexadécatriène-1,6,10 peut être préparé de la manière suivante:

Dans un réacteur de 250 cm$^3$, on introduit 12, 15 g de magnésium, 30 cm$^3$ de tétrahydrofuranne anhydre et un cristal d'iode. On refroidit à -20°C puis on ajoute en 5 heures 30 minutes une solution de 20, 9 g de dichloro-1,7 diméthyl-3,7 octène-2 (mélange des formes E et Z) dans 85 cm$^3$ de tétrahydrofuranne anhydre. On agite pendant 18 heures à -20°C. Le magnésium en excès est séparé par filtration du mélange réactionnel à l'abri de l'air et de l'humidité. La solution obtenue est versée dans une ampoule de coulée placée sur un réacteur contenant 0,5 g d'iodure de cuivre et 5 cm$^3$ de tétrahydrofuranne. On fait couler 1,5 cm$^3$ de la solution magnésienne puis on ajoute rapidement 19,5 g de chloro-3 myrcène dont le titre est de 87% dans 10 cm$^3$ de tétrahydrofuranne.

On refroidit à -20°C puis fait couler la totalité de la solution magnésienne en 3 heures. On laisse ensuite remonter la température au voisinage de 20°C. Le mélange réactionnel est repris par une solution aqueuse de chlorure de sodium puis il est extrait au pentane. La solution pentanique est séchée sur sulfate de magnésium. Après filtration et évaporation du solvant on obtient 29,7 g d'une huile.

L'analyse par chromatographie en phase gazeuse montre que le taux de transformation du chloro-3 myrcène est de 69%.

L'huile obtenue est chauffée à 100-105°C sous pression réduite (0,5-1 mm de mercure; 0,087-0, 13 kPa) afin d'éliminer les produits en $C_{10}$ n'ayant pas réagi. On obtient ainsi un résidu (20,0 g) contenant, d'après les spectres de masse et de résonance magnétique nucléaire du proton, 85% de triméthyl-7,11,15 méthylène-3 chloro- 15 hexadécatriène- 1,6,10.

Le rendement est de 82% par rapport au chloro-3 myrcène consommé.

*Exemple 11*

On opère comme dans l'exemple 2 mais en utilisant:

| | | |
|---|---|---|
| – myrcène (dont la pureté est supérieure à 95%) | 75 | g |
| – iodure cuivreux | 2,62 | g |
| – chlorhydrate de triéthylamine | 1,9 | g |
| – chlorure de méthylène | 150 | cm$^3$ |
| – acide chlorhydrique sec | 20,2 | g |

L'acide chlorhydrique est introduit en 4 heures 30 minutes à une température de -8°C.

Après traitement du mélange réactionnel, on obtient 93,3 g d'une huile dont l'analyse par résonance magnétique nucléaire du proton montre qu'elle est constituée de:

| | |
|---|---|
| – chlorures de géranyle et de néryle | 90% (EZ = 70/30) |
| – chlorures de linalyle | 10% |
| – chlorure de terpényle | traces (inférieur à 0,1%) |
| – myrcène | traces (inférieur à 0,1%) |

Le rendement pondérale est de 98%.

*Exemple 12*

On opère comme dans l'exemple 2 mais en utilisant:

| | | |
|---|---|---|
| – myrcène (dont la pureté est supérieure à 95%) | 75 | g |
| – chlorure cuivreux | 1,36 | g |
| – chlorure de tétra-n. butyl-phosphonium | 4,05 | g |
| – chlorure de méthylène | 150 | cm$^3$ |
| – acide chlorhydrique sec | 20,2 | g |

L'acide chlorhydrique est introduit en 4 heures à une température de -10°C.

Après traitement du mélange réactionnel, on obtient 94,1 g d'une huile dont l'analyse par résonance magnétique nucléaire du proton montre qu'elle et constituée de:

| | |
|---|---|
| – chlorures de géranyle et de néryle | 90% (EZ = 60/40) |
| – chlorures de linalyle | 10% |
| – chlorure de terpényle | traces (inférieur à 0,1%) |
| – myrcène | traces (inférieur à 0,1%) |

Le rendement pondéral est de 99%

*Exemple 13*

Dans un ballon tricol, on introduit, sous atmosphère d'argon, 407 mg de chlorhydrate de triéthylamine, 131 mg de chlorure cuivreux, 6 cm$^3$ de chlorure de méthylène et 6 cm$^3$ d'acide acétique. Après 5 minutes d'agitation à 20°C, on obtient une solution jaune. Après refroidissement à -10°C, on ajoute 13 g de phytatriène à 94% (obtenu selon le brevet américain US 4 292 459) en solution dans 10 cm$^3$ de chlorure méthylène et 10 cm$^3$ d'acide acétique. On introduit alors en 1 heure, à une température comprise entre - 7 et - 10°C, 1,6 g l'acide chlorhydrique anhydre. Après la fin de l'addition d'acide chlorhydrique, on poursuit l'agitation pendant 2 heures à la même température. Le mélange réactionnel est versé dans un mélange de 50 cm$^3$ de pentane et de 50 cm$^3$ d'une solution aqueuse de chlorure d'ammonium à 100 g/litre. Après décantation la phase organique est séchée sur sulfate de sodium. Après filtration et évaporation du solvant on obtient 15,52 g d'une huile dont l'analyse par spectrométrie de résonance magnétique nucléaire du proton et par spectrométrie de masse montre qu'elle est constituée essentiellement de monohydrochlorphytatriène.

L'hydrogénation de 3,5 g de l'huile obtenue en solution dans 30 cm$^3$ d'éthanol en présence de 300 mg de palladium sur noir à 10% en poids sous une pression d'hydrogène de 30 bars pendant 4 heures à 20°C conduit au phytane avec un rendement de 92% et une sélectivité de 98%.

## Revendications

1. Procédé de préparation d'halogénures allyliques primaires et/ou tertiaires par action d'un hydracide halogéné sur un diène conjugué terminal comportant des enchaînements terpéniques en présence d'un catalyseur constitué d'halogénure cuivreux tel que le chlorure ou l'iodure cuivreux associé à un sel d'ammonium quaternaire ou à un sel de phosphonium contenant au maximum 16 atomes de carbone ou à un sel d'amine tertiaire contenant au maximum 10 atomes de carbone caractérisé en ce que l'on effectue la réaction à une température inférieure à 20°C dans un solvant organique capable de solubiliser le système catalytique, puis traite le mélange réactionnel par l'eau et un solvant organique, afin d'éliminer le système catalytique en solution aqueuse, puis sépare les halogénures allyliques primaires et/ou tertiaires après élimination du solvant de la phase organique, et les purifie éventuellement par distillation.

2. Procédé selon la revendication 1 caractérisé en ce que le solvant capable de solubiliser le système catalytique est choisi parmi les hydrocarbures aliphatiques halogénés, les acides organiques, les anhydrides d'acides organiques et leurs mélanges.

3. Procédé selon la revendication 2 caractérisé en ce que le solvant est choisi parmi le chlorure de méthylène, l'acide acétique, l'anhydride acétique et leurs mélanges.

4. Procédé selon la revendication 1 caractérisé en ce que l'on opère à une température inférieure à 0°C.

5. Procédé selon la revendication 1 caractérisé en ce que l'halogénure cuivreux est utilisé dans un rapport molaire compris entre 0,05 et 10 % par rapport au diène conjugué mis en oeuvre.

6. Procédé selon la revendication 1 caractérisé en ce que le sel d'ammonium quaternaire ou le sel de phosphonium est utilisé dans un rapport molaire compris entre 0,05 et 10 % par rapport au diène conjugué mis en oeuvre.

7. Procédé selon la revendication 1 caractérisé en ce que l'hydracide halogéné est l'acide chlorhydrique.

8. Procédé selon la revendication 1 caractérisé en ce que le sel d'ammonium quaternaire ou le sel de phosphonium contenant au maximum 16 atomes de carbone est choisi parmi les halogénures de tétraalcoylammonium et les halogénures de tétraalcoylphosphonium et le sel d'amine tertiaire contenant au maximum 10 atomes de carbone est choisi parmi les halohydrates de trialcoylamines.

9. Procédé selon la revendication 8 caractérisé en ce que le sel d'ammonium quaternaire, le sel de phosphonium et le sel d'amine tertiaire sont choisis parmi les chlorures ou bromures de tétra-n.butylammonium ou de tétra-n.butylphosphonium et le chlorhydrate de triéthylamine.

10. Procédé selon la revendication 1 caractérisé en ce que on lave le mélange réactionnel, à la fin de la phase d'halogénation, par l'eau et un solvant organique choisi parmi les hydrocarbures aliphatiques ou aromatiques puis isole les halogénures allyliques primaires et/ou tertiaires après élimination du solvant.

11. Procédé selon la revendication 1 caractérisé en ce que le diène conjugué terminal comportant des enchaînements terpéniques est choisi parmi le myrcène, le β-farnésène, le β-springène et le triméthyl-7,11,15 méthylène-3 chloro-15 hexadécatriène-1,6,10.

## Patentansprüche

1. Verfahren zur Herstellung von primären und/oder tertiären Allylhalogeniden durch Umsetzung einer Halogenwasserstoffsäure mit einem Terpenketten tragenden endständigen konjugierten Dien, in Gegenwart eines Katalysators, bestehend aus Kupfer(I)-halogenid, z.B, Kupfer(I)chlorid oder -jodid, in Kombination mit einem quaternären Ammoniumsalz oder einem Phosphoniumsalz mit höchstens 16 Kohlenstoffatomen oder einem Salz eines tertiären Amins mit höchstens 10 Kohlenstoffatomen, dadurch gekennzeichnet, daß man die Reaktion bei einer Temperatur unterhalb 20°C in einem organischen Lösungsmittel, das das katalytische System zu solubilisieren vermag, ausführt, dann die Reaktionsmischung mit Wasser und einem organischen Lösungsmittel behandelt, um das katalytische System in wässeriger Lösung zu entfernen, dann die primären und/oder tertiären Allylhalogenide nach Entfernung des Lösungsmittels von der organischen Phase abtrennt und sie gegebenenfalls durch Destillation reinigt.

2. Verfahren nach Anspruch 1, dadurch gekennzeichnet, daß das Lösungmittel, das das katalytische System zu solubilisieren vermag, ausgewählt ist aus den halogenierten aliphatischen kohlenwasserstoffen, den organischen Säuren, den organischen Säureanhydriden und ihren Mischungen.

3. Verfahren nach Anspruch 2, dadurch gekennzeichnet, daß das Lösungsmittel ausgewählt ist aus Methylenchlorid, Essigsäure, Essigsäureanhydrid und deren Mischungen.

4. Verfahren nach Anspruch 1, dadurch gekennzeichnet, daß man bei einer Temperatur unterhalb 0°C arbeitet.

5. Verfahren nach Anspruch 1, dadurch gekennzeichnet, daß das kupfer(I)halogenid in einem molaren Verhältnis zwischen 0,05 und 10 %, bezogen auf eingesetztes konjugiertes Dien verwendet wird.

6. Verfahren nach Anspruch 1, dadurch gekennzeichnet, daß das quaternäre Ammoniumsalz oder das Phosphoniumsalz in einem molaren Verhältnis zwischen 0,05 und 10 %, bezogen auf eingesetztes konjugiertes Dien verwendet wird.

7. Verfahren nach Anspruch 1, dadurch gekennzeichnet, daß die Halogenwasserstoffsäure Chlorwasserstoffsäure ist.

8. Verfahren nach Anspruch 1, dadurch gekennzeichnet, daß das quaternäre Ammoniumsalz oder das Phosphoniumsalz mit höchstens 16 Kohlenstoffatomen ausgewählt ist aus den Tetraalkylammoniumhalogeniden und den Tetraalkylphosphoniumhalogeniden und daß Salz eines tertiären Amins mit höchstens 10 Kohlenstoffatomen ausgewählt ist aus den Trialkylaminhalogenhydraten.

9. Verfahren nach Anspruch 8, dadurch gekennzeichnet, daß des quaternäre Ammoniumsalz, das Phosponiumsalz und das Salz eines tertiären Amins ausgewählt sind aus den Tetra-n-butylammonium- oder Tetra-n-butylphosphoniumchloriden oder -bromiden und Triäthylaminchlorhydrat.

10. Verfahren nach Anspruch 1, dadurch gekennzeichnet, daß man die Reaktionsmischung am Ende der Halogenierungsphase mit Wasser und einem organischen Lösungmittel, ausgewählt aus den aliphatischen oder aromatischen Kohlenwasserstoffen, wäscht, dann die primären und/oder tertiären Allylhalogenide nach Entfernung des Lösungsmittels isoliert.

11. Verfahren nach Anspruch 1, dadurch gekennzeichnet, daß das Terpenketten enthaltende endständige konjugierte Dien ausgewählt ist aus Myrcen, β-Farnesen, β-Springen und 7,11,15-Trimethyl-3-methylen-15-chlor-1,6,10-hexadecatrien.

## Claims

1. A process for the preparation of primary and/or tertiary allyl halides by the action of a halogen hydracid on a terminal conjugated diene containing terpene units in the presence of a catalyst consisting of cuprous halide such as cuprous chloride or iodide combined with a quaternary ammonium salt or with a phosphonium salt containing at most 16 carbon atoms or with a tertiary amine salt containing at most 10 carbon atoms, wherein the reaction is carried out at a temperature less than 20°C in an organic solvent capable of solubilizing the catalytic system, the reaction mixture is then treated with water and an organic solvent in order to remove the catalytic system in aqueous solution, the primary and/or tertiary allyl halides are then separated after removal of the solvent from the organic phase and they are purified optionally by distillation.

2. The process according to Claim 1, wherein the solvent capable of solubilizing the catalytic system is chosen from amongst halogenated aliphatic hydrocarbons, organic acids, organic acid anhydrides and the mixtures thereof.

3. The process according to Claim 2, wherein the solvent is chosen from amongst methylene chloride, acetic acid, acetic anhydride and the mixtures thereof.

4. The process according to Claim 1, wherein the reaction is carried out at a temperature less than 0°C.

5. The process according to Claim 1, wherein the cuprous halide is employed in a molar ratio of between 0.05 and 10% relative to the conjugated diene employed.

6. The process according to Claim 1, wherein the quaternaty ammonium salt or the phosphonium salt is employed in a molar ratio of between 0.05 and 10% relative to the conjugated diene employed.

7. The process according to Claim 1, wherein the halogen hydracid is hydrochloric acid.

8. The process according to Claim 1, wherein the quaternary ammonium salt or the phosphonium salt containing at most 16 carbon atoms is chosen from amongst tetraalkylammonium halides and tetraalkylphosphonium halides and the tertiary amine salt containing at most 10 carbon atoms is chosen from amongst trialkylamine hydrohalides.

9. The process according to Claim 8, wherein the quaternary ammonium salt, the phosphonium salt and the tertiary amine salt are chosen from amongst tetra-n-butyl-ammonium or tetra-n-butylphosphonium chlorides or bromides and triethylamine hydrochloride.

10. The process according to Claim 1, wherein the reaction mixture is washed, at the end of the halogenation phase, with water and an organic solvent chosen from amongst aliphatic or aromatic hydrocarbons and the primary and/or tertiary allyl halides are then isolated after removal of the solvent.

11. The process according to Claim 1, wherein the terminal conjugated diene containing terpene units is chosen from amongst myrcene, β-farnesene, β-springene and 7,11,15-trimethyl-3-methylene-15-chloro-1,6,10-hexadecatriene.

FIGURE 1